# EUROPEAN PATENT APPLICATION

(11) **EP 4 686 488 A1**
(43) Date of publication of application: **04.02.2026**
(21) Application number: 24191787.1
(22) Date of filing: 30.07.2024
(51) Int. Cl.: A61M 5/32, A61M 5/34, A61M 5/50

(54) **DRUG DELIVERY DEVICE LABEL WITH IDENTIFICATION AND TAMPER EVIDENCE FEATURES**

(71) Applicant: Becton, Dickinson and Company, Franklin Lakes, NJ 07417 (US)
(72) Inventor: PETIT, Louis, 38610 Venon (FR); DOLEGA, Lukasz, 38950 Saint-Martin-le-Vinoux (FR); BIANCON, Charles, 38650 MONESTIER DE CLERMONT (FR)
(74) Representative: Germain Maureau

(57) **Abstract**

Provided herein is a drug delivery device (10) that includes a barrel (12) defining a chamber (20) configured to receive a composition, with the barrel comprising a distal tip (28) defining a lumen (30) to provide a passageway for transfer of the composition. The drug delivery device also includes a cylindrical adaptor (50) mounted onto the distal tip and comprising an internal thread defining at least a portion of an inner surface and an outer surface, with the adaptor rotatable relative to the barrel. The drug delivery device further includes a cap (60) mated with the cylindrical adaptor and the distal tip to protect the cylindrical adaptor and the distal tip when the drug delivery device is not in use, and a label (90) applied to at least a portion of the barrel, the adaptor, and at least a portion of the cap, with the label preventing relative rotation between the adaptor and the barrel.

## Description

### BACKGROUND OF THE INVENTION

### Field of the Invention

The present disclosure relates generally to a drug delivery device having an adaptor element and end cap and, more particularly, to a label for the drug delivery device applied over the adaptor element and the end cap to provide additional functionality to the drug delivery device.

### Description of Related Art

Drug delivery devices are used in a variety of environments for administering liquids, such as medications or other drugs, to a patient. One common drug delivery device is a prefilled syringe, which provides the convenience of rapidly delivering the liquid therein to a patient without the need to first aspirate the medication from another container and meter its volume. For dispensing fluids, the prefilled syringe will typically include a syringe barrel that defines a chamber within which the fluid is retained, along with a plunger assembly (including a plunger rod and attached stopper) that is inserted through an open proximal end of the barrel and that is movable within the barrel. Upon a distal actuation of the plunger assembly, fluid may be dispensed out through a tip at the distal end of the syringe barrel.

In some syringes, an adaptor (e.g., a luer lock adaptor) is fitted at the distal end of the syringe barrel and around the tip, with the adaptor configured to connect with a fluid line of a patient. Additionally, a tip cap may be positioned over the tip of the syringe barrel on the distal end and mate with the adaptor, to seal off of the tip and maintain sterility thereof when the syringe is not in use.

With prefilled syringes that include a luer lock adaptor, it may be desirable to prevent the luer lock adaptor from rotating relative to the tip and/or barrel - as rotation of the adaptor relative to the tip/barrel of the syringe may make it difficult for a user to determine whether an external connector is well fitted in the adaptor and, as a consequence, whether the connector is well connected to the tip - as an incorrect connection may cause leaks of product and therefore incorrect doses administered to the patient. Rotation of the adaptor can be prevented by heat shrinking a label or other film to both the adaptor and the syringe barrel.

With prefilled syringes it is also important that integrity of the syringe is ensured, i.e. the integrity of the closure and thus of the fluid filled into the syringe. In some embodiments, ensuring the integrity of the syringe may be achieved by configuring the tip cap as a tamper-evident closure, with the tip cap including a means by which it may be determined whether the syringe has been tampered with, such as by the tip cap having been previously removed. In some embodiments, the tip cap may include a frangible web that is configured to break upon an initial removal of the tip cap, to indicate potential tampering with the syringe. However, it is recognized that incorporation of such tamper-evident features into the tip cap can increase manufacturing costs thereof.

Still further, it is known in the art that it is desirable to have the ability to track the history of individual syringes or lots of syringes from early in the manufacturing life through to the end use of the device. Identification and/or tracking of the syringe may be provided via use of an electronic element that contains data thereon and that can be conventionally read in and read out in a contactless fashion. As one example, an electronic element such as, for example, an RFID tag, may be incorporated into the syringe to provide identification and tracking for the syringe. In some known syringes, the RFID tag is incorporated into the tip cap, such as by being embedded within the tip cap to provide protection thereto, with such embedding of the tag achieved via a co- molding process for forming the tip cap. However, it is recognized that integration of the RFID tag into the tip cap can lead to various problems during manufacturing and/or use of the tip cap, such as the tag leading to adhesion issues in the cap (between the two molded members of the tip cap) and/or limiting the ability to provide torque-transmitting features (e.g., teeth) on the external surface of the tip cap that enable a twist-type removal thereof.

Therefore, it is desirable to provide a film or label that can be applied to an adaptor and a syringe to provide a more secure, anti-rotational connection between the adaptor and the syringe barrel. It is also desirable for such a film or label to be able to provide a tamper-evident feature on the syringe that ensures the integrity of the syringe. It is still further desirable for such a film or label to allow for incorporation of a RFID tag therein, to provide for identification and/or tracking of the syringe.

### SUMMARY OF THE INVENTION

Provided herein is a drug delivery device that includes a barrel defining a chamber configured to receive a composition, with the barrel comprising a distal tip defining a lumen to provide a passageway for transfer of the composition. The drug delivery device also includes a cylindrical adaptor mounted onto the distal tip and comprising an internal thread defining at least a portion of an inner surface and an outer surface, with the adaptor rotatable relative to the barrel. The drug delivery device further includes a cap mated with the cylindrical adaptor and the distal tip to protect the cylindrical adaptor and the distal tip when the drug delivery device is not in use, and a label applied to at least a portion of the barrel, the adaptor, and at least a portion of the cap, with the label preventing relative rotation between the adaptor and the barrel.

In certain configurations, the label comprises an inner surface and an opposed outer surface, and wherein at least a portion of the inner surface comprises an adhesive applied thereto, such that when the label is applied to the at least a portion of the barrel, the adaptor, and the at least a portion of the cap, the inner surface of the label is adhered to an outer surface of the barrel, an outer surface of the adaptor, and an outer surface of the cap.

In certain configurations, the label comprises a shrinkable film that can be made to conform to the outer surface of the barrel, the outer surface of the adaptor, and the outer surface of the cap.

In certain configurations, the label includes a perforated line extending across a width thereof, such that when the label is applied onto the drug delivery device, the perforated line extends circumferentially thereabout, with the perforated line configured to promote tearing of the label along the perforated line.

In certain configurations, with the label applied onto the drug delivery device, the perforated line is positioned at a location aligned with the adaptor.

In certain configurations, removal of the cap from the adaptor and the distal tip requires tearing or removal of the label, such that the label comprises a tamper-evident label that indicates whether the cap has been previously removed.

In certain configurations, the drug delivery device further includes a radio frequency identification (RFID) element positioned on the label, on an inner surface thereof.

In certain configurations, the RFID element comprises a flat RFID tag that conforms to an outer surface of the barrel, the adaptor, and/or the cap, when the label is applied to the drug delivery device.

In certain configurations, the RFID tag has a length in a longitudinal direction of the drug delivery device, with a minimum length of the RFID tag being 1 mm and a maximum length of the RFID tag being a length of the label.

In certain configurations, the label has a length in a longitudinal direction of the drug delivery device, with the length being such that the label covers at least 2 mm of the tip cap and covers at least 1-2 mm of the syringe barrel.

In certain configurations, an outer surface of the label presents a smooth transition between the barrel, the adaptor, and the cap.

In certain configurations, the drug delivery device comprises a syringe.

In certain configurations, the syringe comprises one of a 1 mL syringe, a 1mlL syringe, a 3 mL syringe, a 5 mL syringe, a 10 mL syringe or a 20 mL syringe, where an outer diameter of the adaptor and the cap is less than a diameter of the barrel, equal to a diameter of the barrel, or greater than a diameter of the barrel.

In certain configurations, the adaptor comprises a luer lock access (LLA) adaptor.

Also provided herein is a method of manufacturing a drug delivery device. The method includes mounting the adaptor on the distal tip of the barrel and applying the label to the at least a portion of the barrel, the adaptor, and the at least a portion of the cap.

In certain configurations, the method further includes sterilizing the drug delivery device prior to application of the label.

In certain configurations, in applying the label, a perforated line of the label is positioned at a longitudinal location aligned with the adaptor.

In certain configurations, in applying the label, the label is adhered to the at least a portion of the barrel, the adaptor, and the at least a portion of the cap.

In certain configurations, in applying the label, the label is shrunk so as to generally conform to the at least a portion of the barrel, the adaptor, and the at least a portion of the cap.

In certain configurations, upon application of the label, an outer surface of the label presents a smooth transition between the barrel, the adaptor, and the cap.

### BRIEF DESCRIPTION OF THE DRAWINGS

FIG. 1 is a perspective view of a syringe including a label, according to a non-limiting embodiment described herein;
FIG. 2 is an exploded view of the syringe of FIG. 1;
FIG. 3 is a cross-sectional view of a distal end of the syringe of FIG. 1 taken along line 3-3;
FIG. 4 is a perspective view of the label included in the syringe of FIG. 1, along with a detailed view of a RFID tag provided on the label;
FIG. 5 is a perspective view of the label of FIG. 4, shown in a rolled-up configuration;
FIG. 6 is a side view of a syringe including a label, according to another non-limiting embodiment described herein; and
FIG. 7 is a side view of a syringe including a label, according to another non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

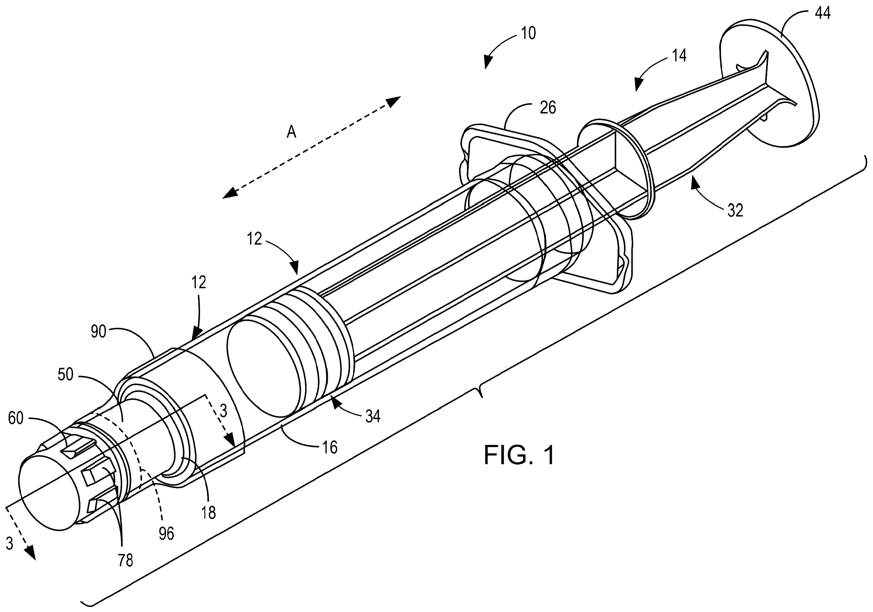

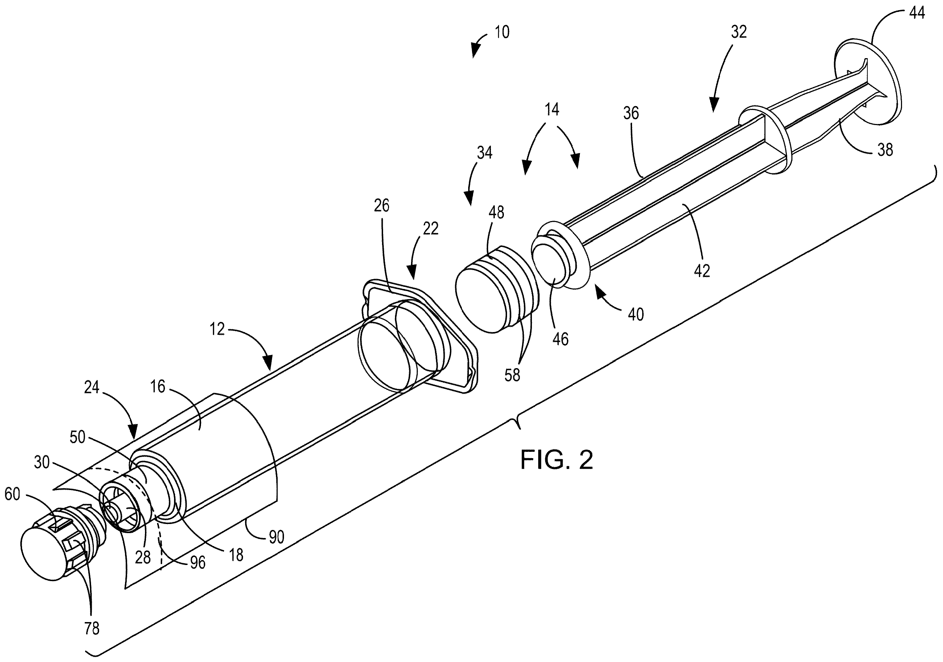

Referring to FIGS. 1 and 2, shown is a non-limiting embodiment of a drug delivery device 10 according to an embodiment of the disclosure. In the illustrated embodiment, the drug delivery device 10 comprises a syringe (hereafter "syringe 10"), such as a prefilled syringe for example, although it is recognized that the drug delivery device 10 is not limited to the illustrated embodiment/construction. The syringe 10 generally includes a syringe barrel 12 and a plunger assembly 14. The plunger assembly 14 is axially movable within the syringe barrel 12 to an advanced position to facilitate administering of an injectable fluid (e.g., medication) to a patient, for example.

The syringe barrel 12 is formed of a generally cylindrical outer wall 16 and an end wall 18 that collectively define a chamber 20 for retaining fluid therein. The syringe barrel 12 includes an open proximal end 22 configured to receive the plunger assembly 14 therein and a distal end 24 at which a shoulder or end wall 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, a tip 28 extends distally outward from the end wall 18 and defines a lumen 30 in fluid communication with the chamber 20, to provide a passageway for the transfer of a fluid out of (or into) the chamber 20. The syringe barrel 12 - including cylindrical outer wall 16, end wall 18, and tip 28 - may be formed of one single material, with the barrel 12 preferably made of glass, although plastic or other known materials may instead be used.

The plunger assembly 14 of syringe 10 is formed of an elongate plunger rod 32 (more generally "plunger 32," as used hereafter) and a plunger head or stopper 34. The plunger 32 may include a main body 36 extending between a plunger proximal end 38 and a plunger distal end 40. In some embodiments, the main body 36 may include a plurality of elongate vanes or walls 42 extending axially along a length thereof between the plunger proximal end 38 and the plunger distal end 40. A thumb press 44 is positioned at the plunger proximal end 38 that may be engaged by a thumb (or other finger) of the user to apply a distally directed force to the plunger assembly 14 to move the plunger 32 with respect to the syringe barrel 12. In some embodiments, a flanged extension member 46 (e.g., disc-shaped flange) is positioned at the plunger distal end 40 that is configured to mate with the stopper 34. In other embodiments, the plunger distal end 40 may include a female receptacle formed therein that is configured to receive and connect to a protrusion (e.g., pin) extending out proximally from the stopper, with the protrusion and receptacle engaging via threading, for example.

The stopper 34 of plunger assembly 14 is positioned at the plunger distal end 40 so as to be movable along with the plunger 32 within the chamber 20 of syringe barrel 12. The stopper 34 may be made from a material that is different from the material of the plunger 32 and that is capable of forming a tight seal with the syringe barrel 12 as it is advanced therethrough. In some embodiments, the stopper 34 includes a receptacle (not shown) formed therein that is sized and configured to receive the flanged extension member 46 of plunger 32, with the flanged extension member 46 coupling with the receptacle via a press fit connection, for example, to secure the stopper 34 to the plunger 32, although it can be appreciated that the stopper 34 and plunger distal end 40 can be secured by other techniques known in the art. The stopper 34 may further include a pair of spaced-apart annular flanges 48 formed thereon (i.e., O-rings) that form a tight seal with the cylindrical outer wall 16 of syringe barrel 12.

As shown in FIGS. 1 and 2, syringe 10 further includes a cylindrical adaptor 50 fitted at the distal end 24 of the syringe barrel 12 and around the tip 28. That is, the adaptor 50 may be mounted on tip 28 by a friction fit, an adhesive, a threaded connection, or other connection known to those of skill in the art. The adaptor 50 may be rotatable relative to the syringe barrel 12 prior to application of a label 90, as described in more detail hereinafter. In some embodiments, the adaptor 50 may abut the end wall 18 of barrel 12, such as by being in physical contact therewith. Alternatively, a small gap may be present between the adaptor 50 and the end wall 18 of the syringe barrel 12. The adaptor 50, along with tip 28, may form a (male) luer lock access connector that is coupleable to a corresponding (female) luer connector to which the syringe 10 is to be brought into engagement, such as the luer connector of a fluid line through which medication is administered to a patient.

Syringe 10 still further includes a tip cap 60 that may be coupled to the tip 28 and the adaptor 50, to protect the tip 28 and adaptor 50 from contamination and/or to maintain the chamber 20 in a sealed condition or state prior to use of the syringe 10. The tip cap 60 may be configured as a rigid cap that is positioned about the tip 28 and extends into a portion of adaptor 50, with the cap configured to provide a fluid-tight seal over the tip 28.

According to aspects of the disclosure, a film or label 90 is applied onto syringe 10 at the distal end thereof. In particular, a label 90 is applied onto syringe 10 so as to be positioned on/over the distal end 24 of syringe barrel 12, the adaptor 50, and least a portion of the tip cap 60. The label 90 may be made of a thermoplastic material such as polyvinyl chloride, polyester, polyethylene, polyamide, polypropylene, vinyl, or some combination thereof, as non-limiting examples, and may be secured to syringe 10 via an adhesive - with the label 90 made to shrink and conform to the syringe 10 after being applied thereto. In some non-limiting embodiments, the label 90 may be made to conform to the syringe 10 by heat-shrinking the label 90, while in other embodiments the label 90 may be made to conform to the syringe 10 by a non-heat-shrinking technique. The label 90 functions to prevent the adaptor 50 from rotating with respect to the syringe barrel 12 and the distal tip 28 around a longitudinal axis A and may also prevent the adaptor 50 from translating lengthwise with respect to the syringe barrel 12.

In applying the label 90 to the syringe 10, the syringe 10 may be sterilized prior to application of label 90. Syringe 10 may be steam sterilized or sterilized using any other process known to those of skill in the art. Label 90 may then be applied to sterilized syringe 10, such as to at least a portion of barrel 12, to adaptor 50, and to at least a portion of tip cap 60.

In some embodiments, label 90 may include one or more indicia printed directly thereon, or indicia may be printed on a sticker (not shown) applied onto the label 90. The indicia may comprise writing such as, for example, graduation, brand name, lot number, expiration date, drug code, or other useful information.

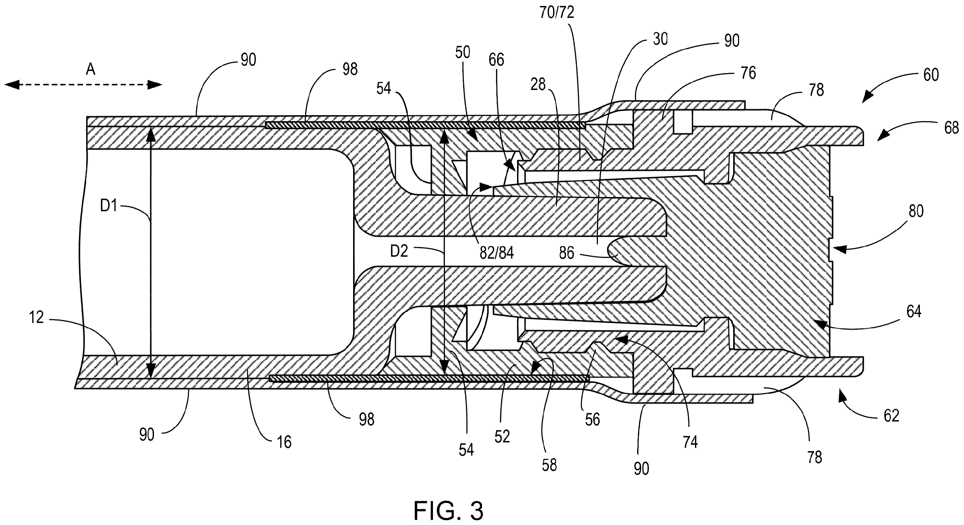

Referring now to FIG. 3, a detailed view of a distal end of the syringe 10 is provided, illustrating the construction and arrangement of adaptor 50, tip cap 60 and label 90.

According to aspects of the disclosure, adaptor 50 defines a ring 52 at a proximal region of the adaptor 50, with an inner projection in the form of a discontinuous annular bulge 54 extending radially inwardly. The adaptor 50 may be made of a flexible material (e.g., plastic) that allows the annular bulge 54 to expand slightly radially in the outward direction under pressure exerted on the inner wall of annular bulge 54. Alternatively, annular bulge 54 may be replaced by a continuous annular bulge capable of expanding radially outwardly. The inner wall or surface 56 of adaptor 50 may be provided with an internal thread defining at least a portion of the inner surface 56, with the internal thread distally spaced from the annular bulge 54. The adaptor 50 may be cylindrical in shape and may have an outer surface 58 that is generally smooth (i.e., free of significant projections of unevenness). The smooth outer surface 58 of adaptor 50 enables the label 90 to be more securely adhered thereto.

In some embodiments, the tip cap 60 is formed as a rigid, molded (or co-molded) cap that may generally be defined as including an outer molded portion 62 and an inner molded portion 64. The outer molded portion 62 includes a first end 66 and a second end 68. The first end 66 includes a connector 70 formed thereon suitable for coupling to the adaptor 50. In some embodiments, connector 70 may thus include a cylindrical portion 72 having a threaded outer surface 74 configured to engage the threaded inner surface 56 of adaptor 50, with the cylindrical portion 72 defining an opening within which the tip 28 of the syringe barrel 12 may be inserted. Adjacent the threaded outer surface 74 of the female connector 70, outer molded portion 62 may include an annular flange 76 that is positioned distally from the first end 66 (and between the first end 66 and the second end 68) such that, upon engagement of the threaded outer surface 74 with the adaptor 50, the annular flange 76 will abut a distal end of the adaptor 50, thereby providing a sealed engagement between the tip cap 60 and the adaptor 50.

The second end 68 of outer molded portion 62 (distal from annular flange 76) is formed as a generally cylindrical, tube-shaped member. In some embodiments, the second end 68 of outer molded portion 62 includes a plurality of spline teeth 78 formed on the outer surface thereof. The spline teeth 78 are spaced circumferentially about the outer molded portion 62 and extend radially outward therefrom, so as to provide an engagement surface with which a twisting tool (i.e., process screwing tool) may engage the tip cap 60 to apply a screwing torque thereto when securing the tip cap 60 to the adaptor 50 - i.e., when engaging the threaded outer surface 74 of the connector 70 on outer molded portion 62 with the threaded inner surface 56 of the adaptor 50.

The inner molded portion 64 of tip cap may be positioned within the outer molded portion 62 and may be formed of a same or different (i.e., softer or more compliant) material than the outer molded portion 62. The inner molded portion 64 includes a closed distal end 80 positioned within the cylindrical second end 68 of outer molded portion 62, along with an open proximal end 82 that extends into the first end 66 of outer molded portion 62. The proximal end 82 of inner molded portion 64 is configured to engage tip 28 of syringe barrel 12, with an outer member 84 receiving the tip 28 therein and an inner protrusion 86 extending into the lumen 30 of tip 28, to seal off the lumen 30.

As shown in FIG. 3, according to an embodiment of the disclosure, label 90 is applied onto syringe 10 so as to extend axially along a length thereof - with the label 90 sized to extend from the distal end 24 of syringe barrel 12, over the adaptor 50, and onto at least a portion of the tip cap 60. That is, label 90 may be applied onto the syringe barrel 12 about the cylindrical outer wall 16 (at the distal end 24 thereof), applied onto the outer surface 58 of adaptor 50, and onto/over the annular flange 76 and outer surface 96 of tip cap 60. According to embodiments, the label 90 should be sized to cover the whole adaptor 50, at least a portion of the tip cap 60, and at least a portion of the syringe barrel 12, with it recognized that it is not necessary that the tip cap 60 is fully covered by the label 90. In order that label 90 is securely attached on the syringe 10, the label 90 should - at minimum - cover at least about 2 mm of the tip cap 60 and about 1 mm to 2 mm of the syringe barrel 12 (1 mm if the syringe barrel 12 is glass or 2 mm if the syringe barrel 12 is plastic, as the label 90 adheres better to glass). As one non-limiting example, the label should have a minimum length of at least 10 mm, so as to cover at least a portion of the tip cap 60, the adaptor 50, and at least a portion of the syringe barrel 12. As a maximum, the label 90 could cover approximately a full length of the syringe 10 - i.e., from a distal end of the tip cap 60 to the proximal end 22 of the syringe barrel 12. As one non-limiting example, such as for a 20 mL syringe, the label 90 could have a maximum length of 120 mm, so as to extend from a distal end of the tip cap 60 to the proximal end 22 of the syringe barrel 12.

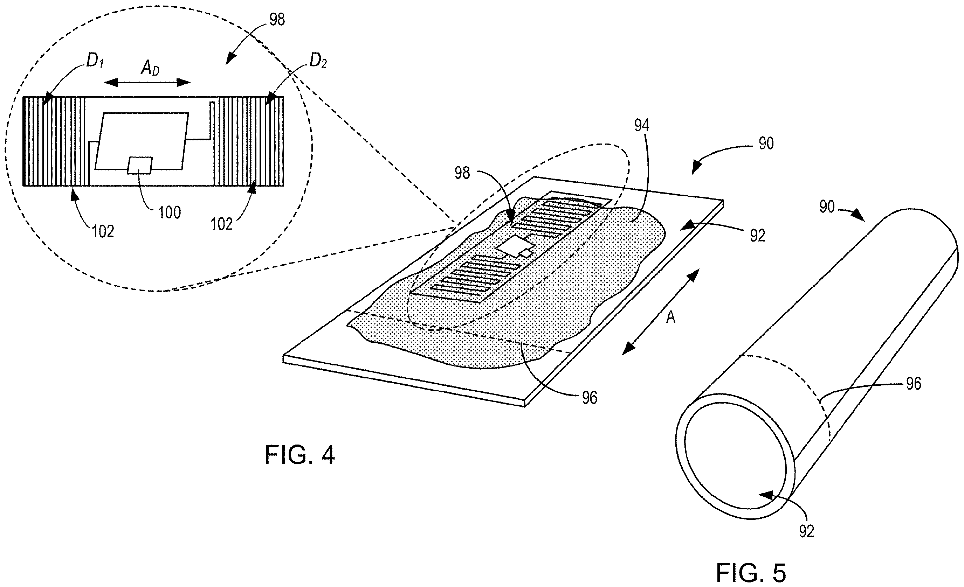

In order to secure the label 90 to the syringe 10, an inward-facing surface 92 of label 90 may have an adhesive material 94 applied over at least a portion thereof, as shown in FIG. 4. FIG. 5 shows label 90 rolled up as if applied to syringe 10. It will be appreciated that adhesive material 94 on inward-facing 92 may adhere label 90 to syringe barrel 12, adaptor 50, and tip cap 60 when label 90 is rolled up and applied to syringe 10. Adhesive material 94 may comprise a glue or be any other type of adhesive suitable for fixedly adhering label 90 to syringe 10.

With the label 90 sized to extend from the distal end 24 of syringe barrel 12, over the adaptor 50, and onto at least a portion of the tip cap 60, the label 90 may provide dual functionality with regards to preventing relative rotation/movement between adaptor 50 and the barrel 12 and providing a means by which it may be determined whether the syringe 10 has been tampered with, such as by the tip cap 60 having been previously removed. First, with the label 90 secured to and extending between the distal end 24 of syringe barrel 12 and the adaptor 50, the adaptor 50 is prevented from moving rotating with respect to the syringe barrel 12 and the tip 28. That is, the label 90 maintains the adaptor 50 blocked in rotation with respect to the syringe barrel 12 around the longitudinal axis A and prevents the adaptor 50 from translating with respect to the syringe barrel 12. Second, with the label 90 secured to and extending between the adaptor 50 and the tip cap 60, the tip cap 60 cannot be removed from the adaptor 50 without breaking or tearing the label, such that the label 90 serves as a tamper-evident feature for the syringe 10.

In some embodiments, and as shown best in FIGS. 4 and 5, label 90 may include a perforated line 96 (or other weakened area or zone) at a desired lengthwise location thereof. The perforated line 96 may extend across an entire width of the label 90, such that when the label 90 is applied onto syringe 10, the perforated line 96 extends circumferentially about the syringe 10. The perforated line 96 may be configured to promote tearing of the label 90 along the line, which may facilitate removal of the tip cap 60 from the adaptor 50 when it is desired to use the syringe 10. In some embodiments, the perforated line 96 is formed on label 90 at a lengthwise location where - with the label 90 applied onto syringe 10 - the perforated line 96 is aligned with the adaptor 50, such as toward a distal end thereof. With the perforated line 96 positioned as such, a distal end of the label 90 may be torn off when the tip cap 70 is removed from the adaptor 50, with a majority of the label 90 remaining intact and secured to the syringe 10. Accordingly, the label 90 is configured as a tamper evident label - as any removal of the tip cap 60 results in separation of the distal portion of the label 90 along perforated line 96, such that even if the tip cap 60 is later repositioned on the syringe 10, the torn label 90 will provide evidence of previous tampering with the syringe 10.

According to aspects and embodiments of the disclosure, the label 90 may also include an RFID element 98 integrated therein that allows for identification and/or tracking of the syringe 10, when the label 90 is secured thereto. That is, the RFID element 98 may include or store information thereon regarding the contents of the syringe 10 (i.e., the medication or drug included therein, if a prefilled syringe) and/or may provide location information to an associated reader, so as to enable tracking of the syringe 10. The RFID element 98 of label 90 may be applied or set onto the inward-facing surface 92 of the label 90, so as to not be visible once the label 90 is applied onto syringe 10. The RFID element 98 may be provided in the form of a generally flat, two-dimensional RFID tag or inlay (hereafter "RFID tag 98"). According to embodiments, the RFID tag 98 may be flexible, so as to conform to the generally cylindrical outer surfaces of the syringe barrel 12, adaptor 50, and tip cap 60 when the label 90 is applied thereto, such that the RFID tag 98 may wrap at least substantially thereabout.

An exemplary construction of the RFID tag 98 is shown in FIG. 4. As shown therein, RFID tag 98 may include an RFID chip 100 connected to an RFID dipole coupling element or antenna 102. The antenna 102 of RFID tag 98 may include two legs or dipole elements D1, D2, with the RFID chip 100 disposed between extremities of the poles D1, D2, such as being centered therebetween. In some embodiments, the RFID tag 98 may be positioned and oriented on label 90 such that a dipole axis, A_{D}, of the RFID tag 98 is approximately aligned with the longitudinal axis A of the syringe 10.

With the label 90 sized to extend from the distal end 24 of syringe barrel 12, over the adaptor 50, and onto at least a portion of the tip cap 60, a lengthwise dimension (and overall size) of the RFID tag 98 may be increased, as compared to if the label 90 extended only between the syringe barrel 12 and adaptor 50. At a maximum end, the RFID tag 98 may be sized to extend approximately an entire length of the label 90, in order to increase the antenna length (and thus the reading length) by a maximum amount. At a minimum end, the RFID tag 98 may be sized to have a length of at least 1 mm. With the RFID tag 98 having such an increased size, a size of the antenna 102 may be increased, which may improve readability of the RFID tag 98 as compared to smaller RFID-tagged labels applied only on a tip cap or luer lock access connector.

Regarding a radial dimension of the label 90 and RFID tag 98, it is recognized that the radial dimension of the label 90 and RFID tag 98 may be bigger than the circumference of the syringe barrel 12, with such a tag size being allowed without interfering with reading of the RFID tag 98 - as the tag may be read in all directions. However, overlapping of the label 90 (when wrapped about the tip cap 60, adaptor 50, and syringe barrel 12) may increase a thickness of the syringe barrel 12 (leading to manutention issues) and/or may lead to adhesion issues with the label 90.

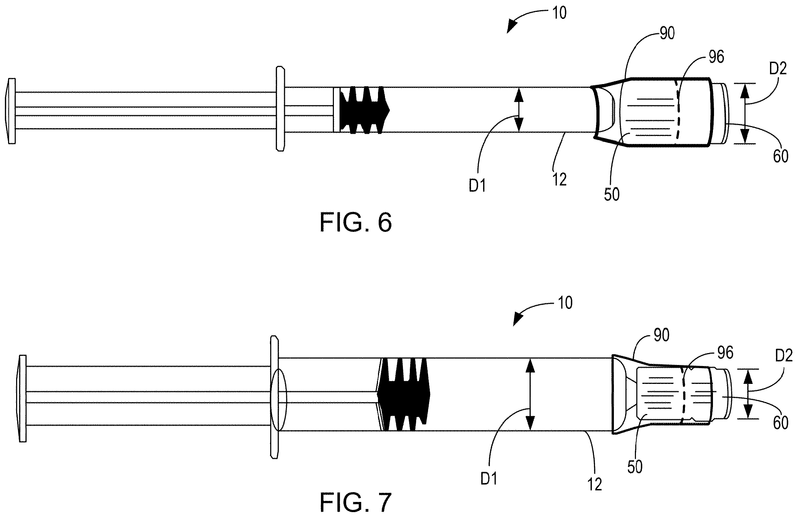

While FIGS. 1-3 are illustrative of a label 90 applied onto a 1 mL syringe where a diameter D1 of barrel 12 is substantially the same as a diameter D2 of adaptor 50 (e.g., within 5%, such as within 1%, or identical to one another), it is recognized that the label 90 may also be suitable for use with other size syringes 10, including 1mlL syringes, 3 mL syringes, 5 mL syringes, 10 mL syringes and/or 20 mL syringes. Non-limiting examples of additional syringe sizes are provided in FIGS. 6 and 7, with a 1mlL syringe (FIG. 6) and a 5 mL syringe (FIG. 7) being shown therein. For the 1mlL syringe, it can be seen that the diameter D1 of the syringe barrel 12 is less than the diameter D2 of adaptor 50 - with the label 90 thus tapering outward from the syringe barrel 12 to the adaptor 50 when applied/shrunk onto the syringe 10, providing a smooth transition therebetween. For the 5 mL syringe, it can be seen that the diameter D1 of the syringe barrel 12 is greater than the diameter D2 of adaptor 50 - with the label 90 thus tapering inward from the syringe barrel 12 to the adaptor 50 when applied/shrunk onto the syringe 10, providing a smooth transition therebetween.

In accordance with aspects of the disclosure, it is recognized that batches of syringes 10 are typically packaged, stored, and/or shipped in nests having numerous chimneys formed therein to hold the syringes. Removal of the syringes 10 from such a nest is performed by axially sliding the syringes 10 relative to the nest and it is possible for a syringe 10 to become lodged within a chimney during extraction from the nest due to diameter differences between the tip cap and syringe barrel. Consequently, an automated processing line associated with the syringes 10, such as for automatically filling the syringes 10 with medication/drugs for example, may need to be stopped until the syringe(s) 10 are able to be dislodged from the nest, leading to undesirable downtime and maintenance costs, as well as potential damage to the syringe(s) 10. According to aspects of the disclosure, inclusion of the label 90 on syringe 10 may function to lessen the likelihood of a syringe 10 becoming lodged within a chimney of the nest during extraction therefrom. That is, as described above, the label 90 may provide a smooth transition between the syringe barrel 12 and the adaptor 50 (and tip cap 60) when applied/shrunk onto the syringe 10 - regardless of the size of the syringe 10 - thus lessening the impact of any diameter difference between the syringe barrel 12 and the adaptor 50 (and tip cap 60), with regard to the syringe 10 becoming caught/lodged in the nest.

Beneficially, embodiments of the invention thus provide a drug delivery device that includes a label that is applied thereon to provide a more secure, anti-rotational connection between the adaptor and the barrel thereof. The label is applied onto each of the barrel, the adaptor, and the cap of the drug delivery device, such that the label also provides a tamper-evident feature on the device that ensures the integrity thereof. The label may include a RFID tag thereon that provides for identification and/or tracking of the drug delivery device, with the dimensions of the label allowing for an RFID tag sized to provide improved readability thereof.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A drug delivery device (10), comprising:
a barrel (12) defining a chamber (20) configured to receive a composition, the barrel comprising a distal tip (28) defining a lumen (30) to provide a passageway for transfer of the composition;
a cylindrical adaptor (50) mounted onto the distal tip, the adaptor comprising an internal thread defining at least a portion of an inner surface and an outer surface, the adaptor rotatable relative to the barrel;
a cap (60) mated with the cylindrical adaptor and the distal tip to protect the cylindrical adaptor and the distal tip when the drug delivery device is not in use; and
a label (90) applied to at least a portion of the barrel, the adaptor, and at least a portion of the cap, with the label preventing relative rotation between the adaptor and the barrel.

2. The drug delivery device (10) of claim 1, wherein the label (90) comprises an inner surface and an opposed outer surface, and wherein at least a portion of the inner surface comprises an adhesive (94) applied thereto, such that when the label is applied to the at least a portion of the barrel (12), the adaptor (50), and the at least a portion of the cap (60), the inner surface of the label is adhered to an outer surface of the barrel, an outer surface of the adaptor, and an outer surface of the cap; and, optionally,
wherein the label comprises a shrinkable film that can be made to conform to the outer surface of the barrel, the outer surface of the adaptor, and the outer surface of the cap.

3. The drug delivery device (10) of claim 1 or claim 2, wherein the label (90) includes a perforated line (96) extending across a width thereof, such that when the label is applied onto the drug delivery device, the perforated line extends circumferentially thereabout, with the perforated line configured to promote tearing of the label along the perforated line; and, optionally,
wherein with the label applied onto the drug delivery device, the perforated line is positioned at a location aligned with the adaptor (50).

4. The drug delivery device (10) of any of claims 1-3, wherein removal of the cap (60) from the adaptor (50) and the distal tip (28) requires tearing or removal of the label (90), such that the label comprises a tamper-evident label that indicates whether the cap has been previously removed.

5. The drug delivery device (10) of any of claims 1-4, further comprising a radio frequency identification (RFID) element (98) positioned on the label (90), on an inner surface thereof; and, optionally,
wherein the RFID element comprises a flat RFID tag that conforms to an outer surface of the barrel (12), the adaptor (50), and/or the cap (60), when the label is applied to the drug delivery device; and, optionally,
wherein the RFID tag has a length in a longitudinal direction of the drug delivery device, with a minimum length of the RFID tag being 1 mm and a maximum length of the RFID tag being a length of the label.

6. The drug delivery device (10) of any of claims 1-5, wherein the label (90) has a length in a longitudinal direction of the drug delivery device, with the length being such that the label covers at least 2 mm of the cap (60) and covers at least 1-2 mm of the barrel (12).

7. The drug delivery device (10) of any of claims 1-6, wherein an outer surface of the label (90) presents a smooth transition between the barrel (12), the adaptor (50), and the cap (60).

8. The drug delivery device (10) of any of claims 1-7, wherein the drug delivery device comprises a syringe; and, optionally,
wherein the syringe comprises one of a 1mL syringe, a 1mlL syringe, a 3 mL syringe, a 5 mL syringe, a 10 mL syringe or a 20 mL syringe, where an outer diameter of the adaptor (50) and the cap (60) is less than a diameter of the barrel (12), equal to a diameter of the barrel, or greater than a diameter of the barrel.

9. The drug delivery device (10) of any of claims 1-8, wherein the adaptor (50) comprises a luer lock access (LLA) adaptor.

10. A method of manufacturing the drug delivery device (10) of any of claims 1-9, comprising:
mounting the adaptor (50) on the distal tip (38) of the barrel (12); and
applying the label (90) to the at least a portion of the barrel, the adaptor, and the at least a portion of the cap (60).

11. The method of claim 10, further comprising sterilizing the drug delivery device (10) prior to application of the label (90).

12. The method of claim 10 or claim 11, wherein in applying the label (90), a perforated line (96) of the label is positioned at a longitudinal location aligned with the adaptor (50).

13. The method of any of claims 10-12, wherein in applying the label (90), the label is adhered to the at least a portion of the barrel (12), the adaptor (50), and the at least a portion of the cap (60).

14. The method of any of claims 10-13, wherein in applying the label (90), the label is shrunk so as to generally conform to the at least a portion of the barrel (12), the adaptor (50), and the at least a portion of the cap (60).

15. The method of any of claims 10-14, wherein upon application of the label (90), an outer surface of the label presents a smooth transition between the barrel (12), the adaptor (50), and the cap (60).
